# EUROPÄISCHE PATENTSCHRIFT

(11) **EP 1 005 477 B1**
(45) Veröffentlichungstag und Bekanntmachung des Hinweises auf die Patenterteilung: **12.06.2002**
(21) Anmeldenummer: 98946410.2
(22) Anmeldetag: 21.08.1998
(51) Int. Cl.: C07H 1/06, C07C 51/47, C07C 55/14, C07C 63/26, C07C 37/82, C07C 201/16

(54) **VERFAHREN ZUR ISOLIERUNG VON ANIONISCHEN ORGANISCHEN SUBSTANZEN AUS WÄSSRIGEN SYSTEMEN MIT KATIONISCHEN POLYMERNANOPARTIKELN**
METHOD FOR ISOLATING ANIONIC ORGANIC SUBSTANCES FROM AQUEOUS SYSTEMS USING CATIONIC POLYMER NANOPARTICLES
PROCEDE PERMETTANT D'ISOLER DES SUBSTANCES ORGANIQUES ANIONIQUES A PARTIR DE SYSTEMES AQUEUX AU MOYEN DE NANOPARTICULES POLYMERES CATIONIQUES

(30) Priorität: 21.08.1997 DE 19736366
(43) Veröffentlichungstag der Anmeldung: 07.06.2000
(73) Patentinhaber: SKW Trostberg Aktiengesellschaft, 83308 Trostberg (DE)
(72) Erfinder: BAYER, Ernst, D-72076 Tübingen (DE); FRITZ, Hans, D-72072 Tübingen (DE); MAIER, Martin, Carlsbad, CA 92008 (US); SCHEWITZ, Jens, D-72076 Tübingen (DE); GERSTER, Michael, D-72072 Tübingen (DE)
(74) Vertreter: HOFFMANN - EITLE
(86) Internationale Anmeldenummer: EP9805340
(87) Internationale Veröffentlichungsnummer: WO9910527

(56) Entgegenhaltungen:
- EP-A- 0 281 390
- FR-A- 2 691 969

## Beschreibung

Die vorliegende Erfindung betrifft ein Verfahren zur Isolierung organischer Verbindungen mit anionischem Charakter aus wäßrigen Systemen unter Verwendung kationischer Polymernanopartikel.

### Hintergrund der Erfindung

Die Isolierung und Charakterisierung anionischer organischer Verbindungen stellt insbesondere in der Umweltanalytik und in Bereichen der Biotechnologie eine große Herausforderung dar. Häufig liegen die zu isolierenden Verbindungen in wäßrigen Systemen in hoher Verdünnung und/oder in Kombination mit anderen Substanzklassen vor. Ein geeignetes Extraktionsverfahren muß daher folgende Anforderungen erfüllen:
- möglichst hohe Selektivität
- keine Notwendigkeit einer vorherigen Derivatisierung der zu isolierenden Substanzen
- effektive Abtrennbarkeit der Extrakte vom wäßrigen System
- einfache Aufkonzentration und Freisetzung der isolierten Substanzen in hoher Reinheit.

Der quantitative Nachweis umweltrelevanter und gesundheitsgefährdender Stoffe in Boden und Wasser gewinnt zunehmend an Bedeutung. So ist es für einige Substanzklassen wie beispielsweise Nitrophenole, Chlorphenole und Phenoxycarbonsäuren, die als Verunreinigungen im Trinkwasser oder Grundwasser vorkommen können, von großem Interesse, nicht nur die Nachweisgrenze der bisher üblichen Verfahren zu unterschreiten, sondern auch Informationen über die exakte Zusammensetzung der Substanzgemische zu erhalten.

Phenole und Phenolderivate werden bisher hauptsächlich als Summenparameter nach DIN 38409 Teil 16 (H16) ermittelt. Diese Bestimmung des Phenolindex erfolgt über eine Farbreaktion mit 4-Aminoantipyrin und gibt keine Auskunft über die Zusammensetzung der Probe sondern nur über deren Gesamtphenolgehalt. Zur Isolierung der Substanzklassen der Nitrophenole und Chlorphenole werden unter anderem auch Festphasenextraktionsverfahren mit RP C₁₈-Phasen angewendet. Dafür ist es jedoch erforderlich, die Proben vor der eigentlichen Extraktion beispielsweise durch die Umsetzung mit Acetanhydrid zu derivatisieren. Dieser zusätzliche Reaktionsschritt kann bei einer unterschiedlichen Reaktivität der einzelnen Phenolderivate zu einer Verfälschung des Analysenergebnisses führen. Ähnliche Probleme treten bei der Extraktion von Phenoxycarbonsäuren auf, die ebenfalls vor der Festphasenextraktion derivatisiert werden müssen.

Auch im Bereich biologischer, pharmazeutischer und medizinischer Anwendungen spielt die Isolierung anionischer organischer Systeme in Form von Peptiden, Nukleinsäuren und Nukleinsäurederivaten eine wichtige Rolle. Beispielsweise stellen synthetische Oligonukleotide mit ihrem polyanionischen Charakter eine neue Klasse von therapeutischen Substanzen dar, die beispielweise in der Antisense- und Antigen-Strategie zur Kontrolle der Genexpression eingesetzt werden. In der Regel werden hierbei modifizierte Nukleinsäurederivate verwendet, die nur aus einer geringen Zahl von Nukleotiden bis zu ca. 25 Nukleotidbausteinen zusammengesetzt sind. Um im Rahmen von präklinischen und klinischen Studien Informationen über den Metabolismus und die Pharmakokinetik dieser Wirkstoffe zu erhalten, ist es erforderlich, die eingesetzten Nukleinsäuren und ihre Abbauprodukte aus biologischen Medien wie Blut, Urin oder Zellextrakten zu isolieren. Nach der Abtrennung sollten die Substanzen in hoher Ausbeute und Reinheit vorliegen und insbesondere keine Verunreinigungen durch Fremdelektrolyte aufweisen, um sie für eine Charakterisierung mittels empfindlicher analytischer Methoden zugänglich zu machen.

Bisher werden Oligonukleotide und ihre Derivate aus biologischen Medien hauptsächlich auf chromatographischem Wege unter Verwendung von Ionenaustauscherharzen extrahiert. Die Elution der Nukleinsäuren erfolgt nach der Abtrennung der Serumbestandteile mit Puffern hoher Ionenstärke. Der hohe Salzgehalt der Proben macht folglich zusätzliche Aufreinigungsschritte zur Entsalzung erforderlich, um eine Charakterisierung des Materials zu ermöglichen. Der Einsatz einer zweistufigen Festphasenextraktion zur Isolierung von Oligonukleotiden aus humanem Blutplasma unter Verwendung einer Kombination von Ionenaustauschchromatographie und Reversed Phase Chromatographie ist in J. M. Leeds, M. J. Graham, L. Truong, L. L. Cummins, Anal. Biochem. 235, 36-43 (1996) beschrieben. Zur Entfernung von Salzrückständen beinhaltet dieses Verfahren als weiteren Aufarbeitungsschritt nach der Extraktion eine Membrandialyse. Erst dann können die Proben zur kapillarelektrophoretischen Analyse eingesetzt werden. Nachteilig bei diesem Verfahren ist die geringe Wiederfindungsrate von ca. 40% und die Tatsache, daß es auf die Isolierung von Oligonukleotiden mit mehr als 15 Nukleotidbausteinen beschränkt ist.

Für einige Anwendungen größerer DNA-Fragmente ist es erforderlich, diese aus komplexen Puffersystemen und enzymhaltigen Lösungen zu extrahieren. So enthält beispielsweise ein PCR-Ansatz neben Polymerasen und Puffersalzen oftmals bestimmte Detergenzien, die zur Stabilisierung des Enzyms erforderlich sind. Klassisch erfolgt die Abtrennung von Nukleinsäuren aus wäßrigen Lösungen mittels Phenolextraktion oder durch Fällung mit Ethanol. Der Einsatz anorganischer Trägermaterialien auf der Basis von Silicagel zur Extraktion von Nukleinsäuren mit einer Länge von 40 Basenpaaren bis 50 Kilobasen ist beispielsweise in WO 9521177-A1 beschrieben. Die adsorptive Anbindung der Nukleinsäuren an die Glasoberfläche erfolgt bei einem pH-Wert kleiner 7,5 in Gegenwart hoher Salzkonzentrationen unter Zusatz eines chaotropen Salzes. Die Freisetzung der gebundenen Nukleinsäuren wird durch eine Verringerung der Ionenstärke erzielt. Die Isolierung eines Plasmids aus Zell-Lysaten unter Verwendung von Extraktionssäulen, die zwei Adsorbentien - ein Ionenaustauscherharz und Silicagel - in zwei unterschiedlichen Segmenten enthalten, ist in DE 4139664-A1 beschrieben. Hierbei werden die zu isolierenden Nukleinsäuren mit einem Puffer niedriger Ionenstärke zunächst auf das Anionenaustauscher-Segment aufgebracht, gewaschen und anschließend mit einem Puffer hoher Ionenstärke auf das Silicagel-Segment eluiert. Nach einem weiteren Waschschritt werden die Nukleinsäuren mit Puffern niedriger Ionenstärke eluiert.

Die Verwendung magnetischer Mikropartikel zur Abtrennung von Polynukleotiden ist beispielsweise in EP 281390-A2 beschrieben. Die Anbindung erfolgt hierbei in detergenshaltigem Phosphatpuffer über die Aminogruppen des Trägermaterials. Nach der magnetischen Abtrennung der beladenen Partikel werden die Nukleinsäuren durch Zusatz von 50% Formamid-haltigem Phosphatpuffer freigesetzt.

### Darstellung der Erfindung

Der vorliegenden Erfindung lag die Aufgabe zugrunde, ein einfaches und kostengünstiges Verfahren zur Isolierung von anionischen organischen Substanzen aus wäßrigen Systemen zu entwickeln. Der Begriff wäßriges System bedeutet hier ein System, in dem Wasser ein Bestandteil, in der Regel der Hauptbestandteil ist, beispielsweise verdünnte Lösungen, biologische Medien und komplexe Puffersysteme. Das Verfahren sollte sowohl für die Extraktion niedermolekularer Verbindungen mit anionischem Charakter wie beispielsweise Phenolderivaten, als auch zur Isolierung hochmolekularer, polyanionischer Substanzen wie beispielsweise Peptiden, Nukleinsäuren und ihrer Derivate geeignet sein.

Das Grundprinzip des Verfahrens basiert auf der Verwendung kationischer Polymernanopartikel mit kovalent gebundenen pH-sensitiven Gruppen, insbesondere Endgruppen. Die gezielte Anbindung der zu isolierenden Substanzen an die Partikel wird durch das Zusammenwirken zweier voneinander unabhängiger Wechselwirkungskräfte, elektrostatischen und hydrophoben Wechselwirkungen, gewährleistet. Die Affinität der vorliegenden Substanzen für die Partikeloberfläche ist sowohl abhängig von ihrem anionischen Charakter als auch von ihrer Hydrophobizität. Diese Kombination ermöglicht durch geeignete Wahl der Extraktions- und Waschbedingungen eine selektive Abtrennung der zu isolierenden Substanzen aus komplexen Gemischen und hochverdünnten Lösungen. Die elektrostatischen Wechselwirkungen werden durch an der Partikeloberfläche vorhandene basische Gruppen bewirkt. Über pH-Wertänderungen des Mediums kann dieser Anteil der Gesamtwechselwirkungskräfte beeinflußt werden. Damit können die an den Partikel gebundenen Substanzen nach ihrer Isolierung durch eine Aufhebung der elektrostatischen Wechselwirkungen gezielt freigesetzt werden. Dies ermöglicht es, ein Verfahren zur Isolierung von anionischen organischen Substanzen aus wäßrigen Systemen zur Verfügung zu stellen, das dadurch gekennzeichnet ist, daß diese Substanzen an kationische Polymernanopartikel reversibel gebunden und nach der Extraktion durch Änderung des pH-Werts des Dispersionsmediums wieder freigesetzt werden.

Erfindungsgemäß wird somit ein Verfahren zur Isolierung von organischen Substanzen mit anionischem Charakter aus wäßrigen Systemen zur Verfügung gestellt, bei dem
- diese Substanzen reversibel an Polymernanopartikel in kationischer, protonierter Form gebunden werden,
- die beladenen Polymernanopartikel vom wäßrigen Ausgangssystem abgetrennt werden und
- die organischen Substanzen von den beladenen Polymernanopartikeln in einem Medium mit einem pH-Wert, bei dem die kationischen Polymernanopartikel deprotoniert werden, wieder freigesetzt werden.

### Wege zur Ausführung der Erfindung

Das erfindungsgemäße Verfahren beinhaltet die Verwendung von Polymerpartikeln mit einer Teilchengröße von 50 nm bis 2 µm, die aus vinylischen Monomerbausteinen wie beispielsweise Styrol oder Styrolderivaten, Acrylsäurederivaten, Methacrylsäurederivaten oder Mischungen dieser aufgebaut sind und zusätzlich basische Gruppen tragen. Die Konzentration der basischen funktionellen Gruppen auf der Partikeloberfläche beträgt vorzugsweise mehr als 0,1 µmol/m².

Die Polymernanopartikel bilden in wäßrigen Medien eine Dispersion. Sie enthalten kovalent gebundene pH-sensitive basische Gruppen, vorzugsweise Endgruppen. Beispiele für solche Gruppen sind stickstoffhaltige Gruppen, insbesondere aromatische oder aliphatische Amino-, Imino- oder Amidino- oder Guanidinogruppen. Die Herstellung der erfindungsgemäß verwendeten Polymernanopartikel kann nach Verfahren erfolgen, wie sie z.B. in WO 98/17317, in J. Colloid and Interface Sci. **195**, 272-288 (1997), in Kolloid-Z. Z.Polym. **239**, 677 (1979) und in Arbeiten von Goodwin et al. (Colloid Polym. Sci., **525**, 464 (1974), Br. Polym. J. **10**, 173 (1978), Colloid Polym. Sci. **257**, 61 (1979)) offenbart sind.

Darüberhinaus ist es im Rahmen der vorliegenden Erfindung möglich, den Polymersuspensionen zur zusätzlichen sterischen Stabilisierung Stabilisatoren in einer Menge von vorzugsweise 0,01 bis 5 Gewichtsprozent bezogen auf den Feststoffgehalt der Suspension zuzusetzen. Als Stabilisatoren werden hierbei vorzugsweise nichtionische Block-Copolymere mit hydrophoben und hydrophilen Anteilen wie beispielsweise Poloxamere oder Poloxamine verwendet.

Die reversible Anbindung der Substanzen erfolgt in einem wäßrigen Ausgangssystem über elektrostatische Wechselwirkungen mit den pH-sensitiven basischen Oberflächengruppen, die gemäß einer bevorzugten Ausführungsform Dissoziationskonstanten von 10⁻⁶ bis 10⁻¹² aufweisen, in Kombination mit hydrophoben Wechselwirkungen mit dem Polymergrundkörper. In der Regel wird das Ausgangssystem hergestellt durch Zugabe der kationischen Polymernanopartikel in Dispersionsform zum wäßrigen System, das die organischen Substanzen mit anionischem Charakter enthält, wobei man wieder eine Dispersion erhält. Das Ausgangssystem kann vor der eigentlichen Konjugatbildung (Beladung der Polymernanopartikel) auf einen gewünschten pH eingestellt werden. Der pH des Ausgangssystems muß in einem Bereich liegen, in dem die Polymernanopartikel in kationischer Form vorliegen und die organische Substanz mindestens teilweise dissoziiert ist.

Die Extraktion der Substanzen aus den entsprechenden Medien erfolgt vorzugsweise bei Temperaturen von 4 bis 60°C und einem pH-Wert kleiner 11. Er kann gegebenenfalls durch Zusatz einer leicht flüchtigen Säure entsprechend eingestellt werden.

Unter dem Begriff "organische Substanz mit anionischem Charakter" (anionische organische Verbindung) werden hier organische Verbindungen verstanden, die in einem wäßrigen System zumindest teilweise in ein oder mehrere Protonen und ein organisches Mono- oder Polyanion dissoziieren. Als anionische organische Verbindungen kommen hierbei beispielsweise Phenole, Phenolderivate, Sulfonsäuren, Carbonsäuren, Aminosäuren, Peptide mit einer oder mehrerer saurer funktioneller Gruppen oder Nukleinsäuren wie beispielsweise Desoxyribonukleotide, Ribonukleotide, chemisch modifizierte Desoxyribonukleotide bzw. Ribonukleotide ab einer Länge von 5 Nukleotideinheiten in Frage.

Die bei der Umsetzung entstehenden Konjugate der Polymerpartikel mit den entsprechenden Substanzen werden gemäß einer bevorzugten Ausführungsform durch z. B. Zentrifugation oder Filtration von den entsprechenden Medien abgetrennt.

Je nach Art der zu isolierenden Substanz und der Zusammensetzung des abzutrennenden Mediums können die abgetrennten Konjugate mit neutralen oder sauren wäßrigen Waschlösungen unterschiedlicher Polarität sowie auch reinem deionisiertem Wasser gewaschen werden. Dieser Aufreinigungsprozeß erfolgt vorzugsweise im Falle der Extraktion von Verbindungen mit polyanionischem Charakter wie beispielsweise Nukleinsäuren aus biologischen Systemen oder komplexen Puffersystemen. Die Resuspendierung der beladenen Polymerpartikel erfolgt gemäß einer bevorzugten Ausführungsform bspw. durch Schütteln oder kurzzeitige Einwirkung von Ultraschall.

Die gezielte Freisetzung (Ablösung) der isolierten und aufgereinigten Substanzen wird dadurch erreicht, daß die Konjugate in ein Dispersionsmedium gegeben werden, dessen pH gegenüber dem Ausgangssystem so geändert ist, daß damit eine Deprotonierung der auf den Partikeln vorhandenen basischen funktionellen Gruppen erreicht wird, was eine Aufhebung der elektrostatischen Wechselwirkungen zwischen den gebundenen Substanzen und den Partikeln zur Folge hat. Eine im wesentlichen vollständige Deprotonierung der kationischen Polymernanopartikel ist zwar bevorzugt, aber nicht unbedingt erforderlich; ein pH, der ausreicht, um die kationischen Polymernanopartikel in einem Umfang zu deprotonieren, so daß die elektrostatischen Wechselwirkungen zumindest im wesentlichen wieder aufgehoben werden, ist ausreichend.

Ein solcher pH wird vorzugsweise durch Zusatz einer leicht flüchtigen Base mit einer Dissoziationskonstante kleiner gleich 10⁻⁹ wie beispielsweise Ammoniak erzielt. Damit wird eine Kontamination der Proben durch Fremdelektrolyt vermieden, so daß die Substanzen nach der Isolierung mit dem erfindungsgemäßen Verfahren in hoher Reinheit vorliegen. Durch Zusatz von geringen Mengen von SDS und Acetonitril beim Ablösungsschritt kann insbesondere bei geringen Konzentrationen der zu isolierenden Substanzen (<1µmol/L) die Empfindlichkeit der Methode zusätzlich erhöht werden.

Gegenüber den bestehenden Techniken zur Isolierung und zur qualitativen und quantitativen Analyse niedermolekularer organischer Substanzen mit anionischem Charakter, wie beispielsweise Phenolderivaten, Carbonsäuren, etc., aus wäßrigen Medien hat das er findungsgemäße Verfahren den wesentlichen Vorteil, daß die zu isolierenden Verbindungen vor der eigentlichen Extraktion nicht derivatisiert werden müssen. Die Anbindung erfolgt durch die Kombination von elektrostatischen und hydrophoben Wechselwirkungen und ermöglicht eine selektive Isolierung von Substanzen, die aufgrund ihrer chemischen Eigenschaften beide Wechselwirkungen mit der Partikeloberfläche ausbilden können. Damit können die Proben auch aus hochverdünnten Lösungen extrahiert werden. Das Prinzip der Festphasenextraktion ermöglicht zudem die einfache Abtrennung der isolierten Substanzen vom umgebenden Medium durch Filtration bzw. Zentrifugation und eine Aufkonzentration der isolierten Proben.

Die besonderen Vorteile des erfindungsgemäßen Verfahrens zur Isolierung von Polyanionen wie z. B. Nukleinsäuren, Peptide, etc. bestehen im wesentlichen darin, daß die Anbindung dieser Stoffe direkt im entsprechenden Medium durchgeführt wird und kein Zusatz von Bindungspuffern erforderlich ist. Dabei lassen sich beispielsweise sowohl kurze natürliche Oligonukleotide bzw. deren Derivate als auch hochmolekulare Polynukleotide direkt aus komplexen wäßrigen Medien unterschiedlicher Zusammensetzung wie beispielsweise Blutplasma bzw. Blutserum, Zellextrakten, Urin oder PCR-Ansätzen isolieren. Aufgrund der starken adsorptiven Wechselwirkungen zwischen Partikel und Polyanionen lassen sich die Konjugate mit Waschlösungen unterschiedlicher Polarität, wie z. B. auch mit deionisiertem Wasser waschen und dadurch auf einfache Weise wirksam entsalzen.

Nach der Aufreinigung der abgetrennten Konjugate erfolgt die Freisetzung der gebundenen Substanzen auf einfache Weise durch pH-Änderung des umgebenden Mediums. Gegenüber den bestehenden Verfahren kann durch die Verwendung leicht flüchtiger Basen eine Kontamination der Proben mit Fremdelektrolyt vermieden werden. Beispielsweise erfordern die bisher entwickelten Verfahren zur Extraktion von Nukleinsäuren den Zusatz von Fremdsalzen, um die Abspaltung des Adsorbats vom Trägermaterial zu ermöglichen. Diese Kontamination erschwert in hohem Maße die analytische Untersuchung der isolierten Substanzen. Demgegenüber hat die hier beschriebene Erfindung den Vorteil, daß die isolierten Nukleinsäuren mit analytischen Methoden wie der Kapillargelelektrophorese (CGE) und der Elektrospray-Massenspektrometrie (ES-MS), die durch ionische Verunreinigungen stark beeinträchtigt werden, charakterisiert werden können. Die Wiederfindungsrate der isolierten Nukleinsäuren beträgt zwischen 50 und 100%.

Überraschenderweise erlaubt das erfindungsgemäße Verfahren im Gegensatz zu bereits bestehenden Techniken trotz mehrfach durchgeführter Waschschritte zur Aufreinigung der Konjugate die Isolierung modifizierter Nukleinsäuren, sowie sehr kurzer Oligonukleotide ab einer Länge von ca. 5 Nukleotidbausteinen. Darüberhinaus ergibt sich eine gewisse Selektivität bei der Anbindung der Nukleinsäuren an die Nanopartikel, die durch längen- und modifikationsabhängige Affinitätsunterschiede verursacht wird.

Das erfindungsgemäße Verfahren stellt daher eine neue und effiziente Methode zur Isolierung von anionischen organischen Substanzen dar. Das Zusammenwirken von elektrostatischen und hydrophoben Wechselwirkungen zwischen den Adsorbatmolekülen und der Partikeloberfläche ermöglicht es, diese auch in hoher Verdünnung bzw. aus komplexen Mischungen zu isolieren. Nach der Isolierung und Aufreinigung lassen sich die gebundenen Substanzen gezielt durch eine pH-Änderung des Mediums freisetzen. Die Extraktion kann direkt aus Medien unterschiedlicher Zusammensetzung erfolgen, ohne daß eine vorhergehende Derivatisierung der Proben bzw. ein Zusatz von Bindungspuffern erforderlich ist.

Die nachfolgenden Beispiele sollen die Erfindung näher veranschaulichen, ohne sie zu begrenzen.

### Beispiel 1:

*Für nachfolgendes Beispiel wird folgende Latexsuspension verwendet: NSI-2-LS, Polystyrolnanopartikel mit ethylenverbrückten Amidiniumendgruppen, stabilisiert mit 0,1% Poloxamer 338 (w*/*v), Durchmesser: 160 nm, Feststoffgehalt: 9,1 g*/*L, Oberflächenkonzentration basischer Gruppen: 0,53 µmol*/*m*^{*2*}*. Die Herstellung der in den Beispielen verwendeten Latices erfolgt nach dem Verfahren gemäß J. Colloid and Interface Sci. 195, (1997) Seite 273.*

200 µL der Latexsuspension NSI-2-LS werden zu 1 mL einer wäßrigen Lösung gegeben, die Adipinsäure in einer Konzentration von 0,1 mmol/L enthält und einen pH-Wert von 7 aufweist. Die Suspension wird unter gelegentlichem Schütteln über einen Zeitraum von 5 min inkubiert. Anschließend werden die erhaltenen Nanopartikel-Adipinsäurekonjugate durch Zentrifugation (24.000 g, 30 min.) vom Überstand abgetrennt. Die Freisetzung der gebundenen Säure erfolgt durch Zugabe von 200 µL 25% igem Ammoniak, wobei gleichzeitig Bernsteinsäure als interner Standard zugegeben wird. Die Menge an isolierter Adipinsäure wird bestimmt, indem die Säuren derivatisiert und anschließend gaschromatographisch quantifiziert werden. Dazu wird zunächst zur ammoniakalischen Probelösung eine methanolische NaOH-Lösung im ca. 4-fach äquivalenten Überschuß hinzugegeben und bei 110°C im Stickstoffstrom zur Trockne abgeblasen, anschließend wird 50 µL Trifluoressigsäure zugegeben und 5 min. bei 110°C inkubiert. Anschließend wird die Lösung bei Raumtemperatur vorsichtig abgeblasen. Die Derivatisierung erfolgt durch Zugabe von 50 µL Bistrimethylsilyltrisfluoroacetamid (BSTFA) bei 60°C in 30 min. Auf gleiche Weise wird zur zusätzlichen Kontrolle die Menge an Adipinsäure im abgetrennten Überstand bestimmt. Die aus 3 Versuchen ermittelte mittlere Menge an adsorbierter Adipinsäure beträgt 2,63 mg/g Polymer. Die Wiederfindungsrate beträgt 33%.

### Beispiel 2:

Analog zu Beispiel 1 wird Terephtalsäure unter Verwendung von 200 µL der Latexsuspension NSI-2-LS aus einer wäßrigen Lösung mit einer Konzentration von 0,1 mmol/L extrahiert. Die aus 3 Versuchen ermittelte Menge an Terephtalsäure beträgt 0,91 mg/g Polymer.

### Beispiel 3:

*Für nachfolgendes Beispiel wird folgende Latexsuspension verwendet: NSI-2-LS, Polystyrolnanopartikel mit ethylenverbrückten Amidiniumendgruppen, stabilisiert mit 0,1% Poloxamer 338 (w*/*v), Durchmesser: 160 nm, Feststoffgehalt: 9,1 g*/*L, Oberflächenkonzentration basischer Gruppen: 0,53 µmol*/*m*^{*2*}.

Eine wäßrige Mischung von 9 unterschiedlichen Phenolderivaten wird zu 200 µL der Latexsuspension NSI-2-LS hinzugeben. Die einzelnen Komponenten der Mischung 2,4-Dinitrophenol, 2-Methyl-4,6-dinitrophenol, 2,5-Dinintrophenol, Phenol, 4-Nitrophenol, 3-Nitrophenol, Pentachlorphenol, 2-Bromphenol, 2,6-Dimethyl-4-nitrophenol, 2-Nitrophenol, 2,4-Dibromphenol liegen in einer Konzentration von 10 µg/ml vor. Die Suspension wird unter gelegentlichem Schütteln über einen Zeitraum von 5 min inkubiert. Anschließend werden die erhaltenen Nanopartikel-Phenolkonjugate durch Zentrifugation (24.000 g, 30 min.) vom Überstand abgetrennt. Die Freisetzung der gebundenen Säure erfolgt durch Zugabe von 200 µL 25% igem Ammoniak. Nach einem weiteren Zentrifugationsschritt (24 000 g, 10 min.) wird das im Überstand befindliche isolierte Nitrophenolgemisch ohne weitere Derivatisierung chromatographisch mittels RP-HPLC analysiert. Als Referenz dient die Ausgangsmischung der Nitrophenole. Die Wiederfindungsraten der einzelnen Komponenten betragen 60-70%. Dieses Beispiel stellt für die Isolierung von niedermolekularen Verbindungen den derzeit besten Ausführungsweg der Erfindung dar.

### Beispiel 4

*Für nachfolgendes Beispiel wird folgende Latexsuspension verwendet: NSI-S1-LS, Polystyrolnanopartikel mit ethylenverbrückten Amidiniumendgruppen, stabilisiert mit 0,1% Poloxamer 338 (w*/*v), Durchmesser: 380 nm, Feststoffgehalt: 10 g*/*L, Oberflächenkonzentration: 0,17 µmol*/*m*^{*2*}.

Zu 200 µL humanem Blutplasma, welches ein Phosphorothioat-Oligonukleotid der Sequenz CTA TTA ACA ACA CAC AAC AG (ODN-1) in einer Konzentration von 100 nmol/L enthält, werden 800 µl 50 mmol/L Tris-HCL (pH9) gegeben. Diese Mischung wird mit 200 µl der Latexsuspension NSI-S1-LS versetzt und unter gelegentlichem Schütteln über einen Zeitraum von 5 min. inkubiert. Die erhaltenen Nanopartikel-Oligonukleotidkonjugate werden durch Zentrifugation (24.000 g, 10 min.) vom Medium abgetrennt und in 1 ml einer Lösung von 0,5 M Essigsäure in Ethanol/Wasser 1:1 (v/v) resuspendiert. Nach der Abtrennung der Waschlösung durch Zentrifugation (24.000 g, 5 min.) werden die Konjugate in 1 ml deionisiertem Wasser resuspendiert und durch nochmalige Zentrifugation abgetrennt. Die Freisetzung des Oligonukleotids erfolgt durch Zugabe von 200 µL 150µmol/L Natriumdodecylsulfat (SDS) in einer Mischung von wäßrigem Ammoniak (25%)/Acetonitril (60:40), wobei gleichzeitig ein dT₃₀-Oligonukleotid als interner Standard zugesetzt wird. Die freigesetzten Oligonukleotide werden durch einen weiteren Zentrifugationsschritt von den Polymerpartikeln abgetrennt. Anschließend wird die Lösung zur Abtrennung evtl. noch in der Lösung vorhandene Partikelrückstände nochmals zentrifugiert. Der Überstand (ca. 80 µL) wird lyophilisiert. Die Wiederfindungsrate wird über Kapillargelelektrophorese bestimmt und beträgt 91%. Dieses Beispiel bildet den derzeit besten Ausführungsweg der Erfindung für polyanionische Verbindungen.

### Beispiel 5

*Für nachfolgendes Beispiel wird folgende Latexsuspension verwendet: NSI-2-LS*.

Analog zu Beispiel 4 wird das Oligonukleotid ODN-1 in unterschiedlicher Konzentration aus humanem Blutplasma unter Verwendung von NSI-2-LS extrahiert, wobei der erste Zentrifugationsschritt 45min. erforderte. Alle nachfolgenden Zentrifugationsschritte erfolgten über einen Zeitraum von jeweils 30 min. Die nachfolgende Tabelle zeigt die über CGE ermittelten Wiederfindungsraten:

| Konzentration | Wiederfindungs rate |
|---|---|
| 100 nmol/L | 89% |
| 25 nmol/L | 84% |
| 10 nmol/L | 88% |
| 5 nmol/L | 64% |

### Beispiel 6

*Für nachfolgendes Beispiel wird die Latexsuspension NSI-2-LS verwendet*.

Analog zu Beispiel 5 wird das Phosphorthioat-Oligonukleotid ODN-2 der Sequenz dT₁₀ (M_{W}: 3125 g/mol) welches in einer Konzentration von 100 nmol/L in humanem Blutplasma vorliegt, unter Verwendung von 200 µL der Latexsuspension NSI-2-LS extrahiert. Die über CGE ermittelte Wiederfindungsrate beträgt 80%.

### Beispiel 7

*Für nachfolgendes Beispiel wird die Latexsuspension NSI-2-LS verwendet*.
200 µL der Latexsuspension NSI-2-LS werden zu 1 ml humanem Blutplasma, welches ein Phosphordiester-Oligonukleotid ODN-3 der Sequenz TTC TTG TCT GCT CTT in einer Konzentration von 4 µmol/L enthält, gegeben und unter gelegentlichem Schütteln über einen Zeitraum von 5 min. inkubiert. Die erhaltenen Nanopartikel-Oligonukleotidkonjugate werden durch Zentrifugation (24.000 g, 30 min.) vom Medium abgetrennt und in einer Lösung von 0,5 M Essigsäure in Ethanol/Wasser 1:1 (v/v) resuspendiert. Nach der Abtrennung der Waschlösung durch Zentrifugation (24.000 g, 30 min.) werden die Konjugate in deionisiertem Wasser resuspendiert und durch nochmalige Zentrifugation abgetrennt. Die Freisetzung des Oligonukleotids erfolgt durch Zugabe von 100 µL 25% igem Ammoniak, wobei gleichzeitig ein dT₂₅-Oligonukleotid (M_{W}: 7543 g/mol) als interner Standard zugesetzt wird. Die freigesetzten Oligonukleotide werden durch einen weiteren Zentrifugationsschritt von den Polymerpartikeln abgetrennt. Anschließend wird die Lösung zur Abtrennung evtl. noch in der Lösung vorhandene Partikelrückstände nochmals zentrifugiert. Der Überstand (ca. 80 µL) wird lyophilisiert. Die Wiederfindungsrate wird über Kapillargelelektrophorese bestimmt und beträgt 86%.

### Beispiel 8

*Für nachfolgendes Beispiel wird die Latexsuspension NSI-2-LS verwendet*.

Analog zu Beispiel 5 wird das 3'-Palmityl-modifizierte Oligonukleotid ODN-4 der Sequenz dT₁₈, welches in einer Konzentration von 10 nmol/L in humanem Blutplasma vorliegt, unter Verwendung von Latex NSI-2-LS extrahiert. Die über CGE ermittelte Wiederfindungsrate beträgt 86%.

### Beispiel 9

*Für nachfolgendes Beispiel wird die Latexsuspension NSI-2-LS verwendet.*
Die Diskriminierung der Nukleinsäuren in Abhängigkeit von ihrer Länge wird an zwei Phosphorthioat-Oligonukleotiden der Sequenz dT₁₀ (ODN-5) und der Sequenz dT₂₀ (ODN-6) untersucht. Hierzu werden 200 µL der Latexsuspension NSI-2-LS mit 1 mL einer 2 µmolaren Lösung der Oligonukleotide in humanem Blutplasma inkubiert und analog Beispiel 7 aufgearbeitet. Die Auswertung über CGE ergibt Wiederfindungsraten von 2% für ODN-5 und 67% für ODN-6.

### Beispiel 10

Analog zu Beispiel 7 werden ein Phosphorothioat-Oligonukleotid der Sequenz dT₁₅ (ODN-7), ein Phosphordiester-Oligonukleotid der Sequenz TTC TTG TCT GCT CTT (ODN-8) und ein 3'-Palmityl-modifiziertes Oligonukleotid der Sequenz TTC TTG TCT GCT CTT (ODN-9), welche in einer Konzentration von 4 µmol/L vorliegen, jeweils aus 1 mL 100%igem fetalem Kälberserum isoliert. Die Wiederfindungsraten werden über CGE bestimmt und betragen 71% für ODN-7, 70% für ODN-8 und 71% für ODN-9.

### Beispiel 11

Analog zu Beispiel 5 werden das Oligonukleotid ODN-1, ODN-4 und ein 2'-O-methylmodifizierter RNA-Strang der Sequenz U₁₈ (ON-1), welche in einer Konzentration von 100 nmol/L vorliegen, aus menschlichem Urin extrahiert. Die Wiederfindungsraten betragen für ODN-1 96%, für ODN-4 72% und für ON-1 83%.

### Beispiel 12

Zur Isolierung eines Plasmids PUC-13 (2.7 kB) aus einem PCR-Ansatz werden 200 µL der plasmidhaltigen Lösung mit 1 mL deionisiertem Wasser verdünnt, mit 200 µL der Polymersuspension NSI-2-LS versetzt und analog zu Beispiel 7 inkubiert und aufgearbeitet, wobei auf den Zusatz eines Standards verzichtet wird. Die Quantifizierung der isolierten Nukleinsäuren erfolgt über die Bestimmung der optischen Dichte bei 260 nm und durch die densitometrische Auswertung der Banden der gelelektrophoretischen Analyse der Proben vor und nach der Extraktion. Die ermittelte Wiederfindungsrate beträgt ca. 50%.

## Patentansprüche

1. Verfahren zur Isolierung von organischen Substanzen mit anionischem Charakter aus wäßrigen Systemen, bei dem
- diese Substanzen reversibel an Polymernanopartikel in kationischer, protonierter Form gebunden werden,
- die beladenen Polymernanopartikel vom wäßrigen Ausgangssystem abgetrennt werden und
- die organischen Substanzen von den beladenen Polymernanopartikeln in einem Medium mit einem pH-Wert, bei dem die kationischen Polymernanopartikel deprotoniert werden, wieder freigesetzt werden.

2. Verfahren nach Anspruch 1, **dadurch gekennzeichnet, daß** die Polymernanopartikel pH-sensitive basische Oberflächengruppen mit Dissoziationskonstanten von 10⁻⁶ bis 10⁻¹² tragen.

3. Verfahren nach Anspruch 1 oder 2, **dadurch gekennzeichnet, daß** die pH-sensitiven basischen Gruppen auf der Partikeloberfläche in einer Konzentration von mehr als 0,1 µmol/m² vorliegen.

4. Verfahren nach wenigstens einem der vorhergehenden Ansprüche, **dadurch gekennzeichnet, daß** die organischen Substanzen mit anionischem Charakter im Schritt der Bindung an die kationischen Polymernanopartikel als Mono- oder Polyanionen vorliegen und ausgewählt werden aus Phenolen, Phenolderivaten, Sulfonsäuren, Sulfonsäurederivaten, Carbonsäuren, Phosphorsäurederivaten, Aminosäuren bzw. Peptiden mit einer oder mehreren sauren funktionellen Gruppen, oder Nukleinsäuren wie z.B. Desoxyribonukleotiden, Ribonukleotiden oder chemisch modifizierten Desoxyribonukleotiden bzw. Ribonukleotiden ab einer Länge von 5 Nukleotideinheiten.

5. Verfahren nach wenigstens einem der vorhergehenden Ansprüche, **dadurch gekennzeichnet, daß** die Beladung der kationischen Polymernanopartikel bei Temperaturen von 4 bis 60°C und einem pH-Wert kleiner 11 erfolgt.

6. Verfahren nach wenigstens einem der vorhergehenden Ansprüche, **dadurch gekennzeichnet, daß** die beladenen Polymernanopartikel durch Zentrifugation oder Filtration vom wäßrigen Ausgangssystem abgetrennt werden.

7. Verfahren nach wenigstens einem der vorhergehenden Ansprüche, **dadurch gekennzeichnet, daß** die vom Ausgangssystem abgetrennten beladenen Polymernanopartikel durch Behandlung mit neutralen oder sauren wäßrigen Waschlösungen unterschiedlicher Polarität oder reinem Wasser aufgereinigt werden, bevor die Freisetzung der gebundenen Substanzen erfolgt.

8. Verfahren nach wenigstens einem der vorhergehenden Ansprüche, **dadurch gekennzeichnet, daß** die gebundenen Substanzen nach ihrer Abtrennung und gegebenenfalls ihrer Aufreinigung durch Zusatz einer Base freigesetzt werden.

9. Verfahren nach Anspruch 8, **dadurch gekennzeichnet, daß** als Base eine leicht flüchtige Substanz mit einer Dissoziationskonstante kleiner gleich 10⁻⁹, wie z.B. eine wäßrige Ammoniaklösung verwendet wird.

10. Verfahren nach wenigstens einem der vorhergehenden Ansprüche, **dadurch gekennzeichnet, daß** die Freisetzung in einem wäßrigen Medium erfolgt, das zusätzlich anionische Tenside und/oder Acetonitril enthält.

11. Verfahren nach wenigstens einem der vorhergehenden Ansprüche, **dadurch gekennzeichnet, daß** die Polymernanopartikel durch Zusatz von 0,01 bis 5 Gew.% Stabilisatoren, bezogen auf den Feststoffgehalt einer Polymernanopartikel - Suspension, stabilisiert sind.

## Claims

1. Process for isolating organic substances having anionic character from aqueous systems, in which
- these substances are bound reversibly on polymer nanoparticles in cationic, protonised form,
- the charged polymer nanoparticles are separated off from the aqueous starting system, and
- the organic substances are freed again of the charged polymer nanoparticles in a medium having a pH value, in which the cationic polymer nanoparticles are deprotonised.

2. Process according to claim 1, **characterised in that** the polymer nanoparticles carry pH-sensitive basic surface groups having dissociation constants of 10⁻⁶ to 10⁻¹².

3. Process according to claim 1 or 2, **characterised in that** the pH-sensitive basic groups are present on the particle surface in a concentration of more than 0.1 µmole/m².

4. Process according to at least one of the preceding claims, **characterised in that** the organic substances having anionic character are present in the step of binding to the cationic polymer nanoparticles as monoanions or polyanions and are selected from phenols, phenol derivatives, sulphonic acids, sulphonic acid derivatives, carboxylic acids, phosphoric acid derivatives, amino acids or peptides having one or more acid functional groups, or nucleic acids, such as for example deoxyribonucleotides, ribonucleotides or chemically modified deoxyribonucleotides or ribonucleotides from a length of 5 nucleotide units.

5. Process according to at least one of the preceding claims, **characterised in that** charging of the cationic polymer nanoparticles takes place at temperatures from 4 to 60°C and a pH value less than 11.

6. Process according to at least one of the preceding claims, **characterised in that** the charged polymer nanoparticles are separated off from the aqueous starting system by centrifuging or filtration.

7. Process according to at least one of the preceding claims, **characterised in that** the charged polymer nanoparticles separated off from the starting system are purified by treatment with neutral or acid aqueous washing solutions of different polarity or pure water, before release of the bound substances takes place.

8. Process according to at least one of the preceding claims, **characterised in that** the bound substances are released after their separation and optionally their purification by adding a base.

9. Process according to claim 8, **characterised in that** a highly volatile substance having a dissociation constant less than 10⁻⁹, such as for example an aqueous ammonia solution, is used as the base.

10. Process according to at least one of the preceding claims, **characterised in that** the release takes place in an aqueous medium additionally containing anionic surfactants and/or acetonitrile.

11. Process according to at least one of the preceding claims, **characterised in that** the polymer nanoparticles are stabilised by adding 0.01 to 5 wt.% of stabilisers, based on the solids content of a polymer nanoparticle suspension.

## Revendications

1. Procédé d'isolement de substances organiques à caractère anionique à partir de systèmes aqueux, selon lequel
- on lie de façon réversible ces substances à des nanoparticules de polymère sous forme protonée cationique,
- on sépare les nanoparticules de polymère chargées du système aqueux de départ et
- on libère de nouveau les substances organiques à partir des nanoparticules de polymère chargées dans un milieu ayant un pH auquel les nanoparticules de polymère cationiques sont déprotonées.

2. Procédé selon la revendication 1, **caractérisé en ce que** les nanoparticules de polymère portent des groupes de surface basiques sensibles au pH, ayant des constantes de dissociation comprises entre 10⁻⁶ et 10 ⁻¹².

3. Procédé selon la revendication 1 ou 2, **caractérisé en ce que** les groupes basiques sensibles au pH se trouvent sur la surface des particules à une concentration supérieure à 0,1 µmol/m².

4. Procédé selon au moins l'une des revendications précédentes, **caractérisé en ce que**, dans l'étape de liaison aux nanoparticules de polymère cationiques, les substances organiques à caractère anionique se trouvent sous forme de mono- ou polyanions et sont choisies parmi des phénols, des dérivés de phénol, des acides sulfoniques, des dérivés d'acides sulfoniques, des acides carboxyliques, des dérivés d'acide phosphorique, des aminoacides ou des peptides ayant un ou plusieurs groupes acides fonctionnels, ou des acides nucléiques comme, par exemple, des désoxyribonucléotides, des ribonucléotides ou des désoxyribonucléotides ou ribonucléotides chimiquement modifiés, ayant une longueur d'au moins 5 unités nucléotidiques.

5. Procédé selon au moins l'une des revendications précédentes, **caractérisé en ce que** la charge des nanoparticules de polymère cationiques s'effectue à des températures de 4 à 60°C et à un pH inférieur à 11.

6. Procédé selon au moins l'une des revendications précédentes, **caractérisé en ce que** l'on sépare les nanoparticules de polymère chargées du système aqueux de départ par centrifugation ou par filtration.

7. Procédé selon au moins l'une des revendications précédentes, **caractérisé en ce que** l'on purifie les nanoparticules de polymère chargées séparées du système de départ par traitement avec des solutions de lavage aqueuses neutres ou acides de polarité différente ou avec de l'eau pure avant d'effectuer la libération des substances liées.

8. Procédé selon au moins l'une des revendications précédentes, **caractérisé en ce que**, après leur séparation et éventuellement leur purification, on libère les substances liées par addition d'une base.

9. Procédé selon la revendication 8, **caractérisé en ce que** l'on utilise comme base une substance volatile ayant une constante de dissociation inférieure à 10⁻⁹ comme, par exemple, une solution aqueuse d'ammoniac.

10. Procédé selon au moins l'une des revendications précédentes, **caractérisé en ce que** la libération s'effectue dans un milieu aqueux qui contient en outre des agents tensioactifs anioniques et/ou de l'acétonitrile.

11. Procédé selon au moins l'une des revendications précédentes, **caractérisé en ce que** les nanoparticules de polymère sont stabilisées par l'addition de 0,01 à 5 % en masse de stabilisants par rapport à la teneur en matière solide d'une suspension de nanoparticules de polymère.
